Europäisches Patentamt

European Patent Office

Office européen des brevets

(11) Publication number: **0 288 243**
**A2**

# EUROPEAN PATENT APPLICATION

(21) Application number: 88303511.5

(22) Date of filing: 19.04.88

(51) Int. Cl.⁴: **C07K 7/10 , C07K 5/00 ,**
**A61K 37/48**

(30) Priority: 21.04.87 GB 8709400

(43) Date of publication of application:
**26.10.88 Bulletin 88/43**

(84) Designated Contracting States:
**AT BE CH DE ES FR GB GR IT LI LU NL SE**

(71) Applicant: **E.R. SQUIBB & SONS, INC.**
**Lawrenceville-Princeton Road**
**Princeton New Jersey 08543-400(US)**

(72) Inventor: **Smith, Anthony David, Prof.**
**27 Abberbury Road**
**Oxford OX4 4ET(GB)**
Inventor: **Greenfield, Susan Adele, Dr.**
**Lincoln College**
**Oxford OX1 3DE(GB)**

(74) Representative: **Pennant, Pyers et al**
**Stevens, Hewlett & Perkins 5 Quality Court**
**Chancery Lane**
**London, WC2A 1HZ(GB)**

(54) Acetylcholinesterase.

(57) Acetyloholinesterase (AChE) exists in several forms, some of which are not membrane bound and have other than cholinergic functions. The invention involves the use of AChE or variants or fragments in the treatment of disorders of the central nervous system e.g. Alzheimers and Parkinsons Disease. Administration may be via the cerebrospinal fluid. Among various polypeptides for this purpose, Pro-Glu-Pro and Arg-Pro-Glu-Pro have been synthesised and characterised. Pro-Glu-Pro causes hyperpolarisaton of presumed dopaminergic cells in the substantia nigra in slices of guinea pig brain.

EP 0 288 243 A2

# ACETYLCHOLINESTERASE

This invention concerns the use of acetylcholinesterase and related enzymes and fragments thereof in the treatment of disorders of the central nervous system. There are a number of diseases, including Parkinsons disease and senile dementia (of which Alzheimers disease is the most common form) which involve dysfunction, disappearance or degeneration of cells in particular locations within the brain. There is a need for substances which could cure or alleviate the symptoms of these diseases, or arrest the degeneration of cells.

Parkinsons disease involves degeneration or loss of nerve cells in the substantia nigra, resulting in loss of control over voluntary movements. These cells release dopamine in the striatum and the cortex, and their degeneration results in reduced concentrations of dopamine in the substantia nigra and the striatum.

Alzheimers disease is characterized by deposits of abnormal proteins in the cortex; the deposits take two forms: tangles are masses of dark filaments in the cytoplasm of nerve cells; plaques are patches of degenerating nerve terminals surrounding a dense core. All current attempts at therapy are based on drugs that act on cholinergic synapses. However, Alzheimers disease is a multi-system degenerative disease not confined to the cholinergic system: in addition to the loss of neurons in the cholinergic nucleus basalis, there is damage to, or a loss of, neurons in the raphe nuclei and in the locus coeruleus. These three subcortical nuclei all have extensive projections into the limbic system and neocortex. The raphe nuclei are responsible for the release of 5-hydroxytryptamine in the cortex. The locus coeruleus is responsible for the release of noradrenalin in the cortex.

The classical view of acetylcholinesterase (AChE) is that it is a membrane-bound enzyme exclusively involved in the hydrolysis of acetylcholine. More recently, there have been reports in the literature to the effect that there are least five isoenzymes which are not membrane-bound, and which have functions other than the traditional cholinergic role. The following evidence is relevant.

## 1. AChE is a neurosecretory protein

A soluble isoenzyme of (AChE) has been shown to be secreted into the CSF or into perfusates from discrete brain areas in several different species in response to physiological stimuli: rat, cat, rabbit (Chubb et al., 1976; Greenfield & Smith, 1979; Greenfield et al. 1980; Weston & Greenfield, 1986; Vogt et al, 1984). One site of origin of this secretory isoenzyme is the striatum and another is the substantia nigra (Greenfield et al., 1980). Secretion from the substantia nigra has been studied in the most detail and it shows properties similar to the release of dopamine from the nigra, e.g. the release is resistant to tetrodotoxin but requires calcium ions (Cuello et al. 1981; Weston and Greenfield, 1986). It is very likely therefore that AChE is secreted from the dendrites and/or cell bodies of dopamine-containing neurons in the substantia nigra by mechanisms quite different from those used in transmitter release at nerve endings (reviewed by Greenfield, 1985).

## 2. AChE is altered in senile dementia

The levels of this isoenzyme of AChE are markedly low in the ventricular CSF of patients with Alzheimers disease (Appleyard et al., 1983), but are not significantly different from controls in the lumbar CSF of these patients (Appleyard et al., 1987). This indicates that there is a deficient secretion of AChE from neurons in the brain but not from neurons in the spinal cord in Alzheimers disease.

The nucleus basalis, the raphe nuclei and the locus coeruleus are all characterized by being rich in AChE (Smith and Cuello, 1984). It has since been reported that the somatostatin neurons of the human neocortex, which degenerate in Alzheimers disease, also contain AChE (Nakaumura and Vincent, 1985).

### 3. Non-cholinergic actions of AChE

Unexpected biological effects of the secretory isoenzyme of AChE have been demonstrated both in vivo and in vitro in animals.

(a) A single application of AChE to the substantia nigra unilaterally leads to circling behaviour in rats, which persists for as long as three weeks (Greenfield et al., 1984).

(b) Bilateral administration into the substantia nigra on both sides leads to stereotyped behaviour in rats (Weston & Greenfield, 1985).

(c) Administration of AChE into the cisternal CSF of rats which had previously received a unilateral lesion of the dopamine pathway from the nigra attenuated the lesion-induced circling behaviour for several days (Greenfield et al., 1986). It thus appears that AChE can activate a compensatory neuronal system.

(d) Application of AChE to the nigra modulates the response of dopamine neurons to the effects of stimulation of the striatum. Low doses enhances the response to striatal stimulation whereas high doses inhibit the response (Last and Greenfield, 1987).

(e) In vitro studies with brain slices have shown that AChE opens potassium channels in dopamine neurons in the nigra (Greenfield et al., 1987). This has a modulatory effect since it can result either in increased responsiveness to excitatory inputs by activating low threshold calcium channels in dendrites, or it could lead to inhibition at the level of the cell body.

All the above responses to AChE are unrelated to its catalytic activity since they are not replicated by equal or higher catalytic activities of butyrylcholinesterase and, in the slice experiments, a sample of AChE that had been irreversibly inhibited by Soman was just as effective as untreated AChE. It is clear therefore that a novel biological activity resides in the molecule of AChE which is quite unrelated to its action on cholinergic transmission.

The sequence of the catalytic subunit of AChE purified from Torpedo californica has been published (Schumacher et al., 1986). Although this is derived from the asymmetric form, it is very likely that the sequence of the secretory isoenzyme (globular G4 form) will show a high degree of homology (Massoulie & Bon, 1982; Zevin-Sonkin et al, 1985).

We believe that AChE, in particular the secretory isoenzyme, may have actions on the AchE-containing systems which degenerate in brain disease, similar to its actions in the substantia nigra; and that replacement of lost AChE in these systems may provide some compensation for the loss of neurons, firstly by modulation of the excitability of the remaining neurons and, secondly, by direct effects on target neuronal systems.

In one aspect the invention provides a substance for the treatment of disorders of the central nervous system, which substance is acetylcholinesterase (AChE) or a variant or fragment thereof having the biological action of ACRE. The AChE or variant or fragment thereof preferably shows neuromodulatory activity which is preferably not confined to cholinergic systems.

It is believed that the use of an enzyme or variant or fragment thereof to treat disorders of the central nervous system is completely new. As noted above, one form of the AChE enzyme has been sequenced (Schumacher et al 1986). Although the five or more isoenzymes found in human tissue differ in certain respects, these are expected to be minor: there may be minor conservative substitution of amino acids in a few places; or the differences may lie in polysaccharide chains associated with the polypeptide. Although one soluble isoenzyme has been shown to have considerable neuromodulatory activity not confined to cholinergic systems, it is likely that other isoenzymes will show the same activity to a lesser extent.

We further believe that the neuromodulatory activity may reside in a particular part of the AChE molecule, and that it may be equivalent to one of the peptides produced by limited proteolysis by enzymes similar to those involved in the processing of other pro-proteins in the nervous system which yield biologically active peptides.

The fact that AChE itself displays carboxyl esterase activity on certain peptides and proteins (Small et al, 1986) may mean that some of the processing is autocatalysed. The AChE published sequence contains seven sets of double basic amino acids, i.e. potential sites of cleavage by a trypsin-like pro-protein converting enzyme (Loh et al, 1984). These cleavage sites are:-

-At the amino end of the sequence Arg-Arg; Lys-Lys; Arg-Lys; Lys-Arg; or possibly Gln-Ala.

-At the carboxyl end Arg-Arg; Lys-Lys; Arg-Lys; Lys-Arg; Gly-Arg; or Gly-Lys. In the last two cases the final carboxyl group of the polypeptide is amidated.

It is likely that one or more of the seven polypeptides defined by these potential cleaving sites may show neuromodulatory activity and/or value in the treatment of neurological disease. This invention envisages these seven polypeptides as new compounds, and as substances for the treatment of neurological disease. The polypeptides have the amino acid sequences set out below, any carboxylic acid groups being optionally esterified or amidated;

-The tripeptide Pro-Glu-Pro, in which the gamma carboxylic acid group of Glu may be free, esterified or amidated. This tripeptide appears at numbers 182 to 190 of the nucleotide sequence published by Schumacher et al.

-Pro-Trp-Ser-Gly-Val-Trp-Asn-Ala-Ser-Thr-Thr-Pro-Asn-Asn-Cys-Gln-Gln-Tyr-Val-Asp-Glu-Gln-Phe-Pro-Gly-Phe-Ser-Gly-Ser-Glu-Met-Trp-Asn-Pro-Asn-Arg-Glu-Met-Ser-Glu-Asp-Cys-Leu-Tyr-Leu-Asn-Ile-Trp-Val-Pro-Ser-Pro-Arg-Pro-Lys-Ser-Thr-Thr-Val-Met-Val-Trp-Ile-Tyr-Gly-Gly-Gly-Phe-Tyr-Ser-Gly-Ser-Ser-Thr-Leu-Asp-Val-Tyr-Asn-NH₂. In this sequence the two cysteine residues may be in the thiol form or condensed together to give a disulphide bridge. This polypeptide appears at numbers 197 to 435 of the published nucleotide sequence.

-Ala-Ile-Leu-Gln-Ser-Gly-Ser-Pro-Asn-Cys-Pro-Trp-Ala-Ser-Val-Ser-Val-Ala-Glu-NH₂. This polypeptide appears at numbers 704 to 760 of the published nucleotide sequence. It may be extended at the carboxyl end by Gly.

-Ala-Val-Glu-Leu. This polypeptide appears at numbers 773 to 784 of the published polynucleotide sequence.

-Asn-Leu-Asn-Cys-Asn-Leu-Asn-Ser-Asp-Glu-Glu-Leu-Ile-His-Cys-Leu-Arg-Glu. In this sequence the two cysteine residues may be in the thiol form or condensed together to give a disulphide bridge. This polypeptide appears at numbers 793 to 844 of the published polynucleotide sequence.

-Pro-Gln-Glu-Leu-Ile-Asp-Val-Glu-Trp-Asn-Val-Leu-Pro-Phe-Asp-Ser-Ile-Phe-Arg-Phe-Ser-Phe-Val-Pro-Val-Ile-Asp-Gly-Glu-Phe-Phe-Pro-Thr-Ser-Leu-Glu-Ser-Met-Leu-Asn-Ser-Gly-Asn-Phe. This polypeptide appears at numbers 851 to 982 of the published polynecleotide sequence.

-Asp-Asp-His-Ser-Glu-Leu-Leu-Val-Asn-Thr-Lys-Ser-NH₂. This polypeptide appears at numbers 41 to 76 of the published polynecleotide sequence. It may be extended at the carboxyl end by Gly.

As is well known, in some instances substitution of one amino acid for another, e.g. of Val for Leu, known as conservative substitution, can take place without signficantly affecting the properties of the polypeptide. This may involve substitution of one neutral amino acid and residue by another; or substitution of a charged amino acid residue by another carrying the same charge; or substitution of an amino acid residue with a free carboxy group by one in which the carboxy group is amidated, or vice versa. It is also known that replacement of one or more residues by the D-form of the amino acid or a related amino acid might enhance or block the biological activity. The invention extends to polypeptides in which such substitution has taken place. The possibility exists that any of the polypeptide listed may have an additional basic amino acid residue (ie Lys or Arg) at either the carboxy terminal end or the amino terminal end, or both. This is because the natural cleavage enzymes initially split the peptide bond between two basic amino acids, and it cannot necessarily be assumed in every case that the single basic amino acid left attached to the polypeptide will necessarily be removed. The invention extends to polypeptides with one or two such terminal basic amino acid residues.

Isoenzymes of AChE can be prepared and purified by conventional techniques from various sources, e.g. foetal calf serum. Electric eel AChE is available commercially. Polypeptides can be prepared by proteolysis of the enzyme or via the DNA by genetic engineering techniques. Peptides containing limited numbers of amino acids can be prepared by standard synthetic routes. The invention also encompasses the DNA sequences which code for the polypeptides described.

In another aspect, the invention concerns a composition for administration to a patient suffering from a disorder of the central nervous system comprising a substance or polypeptide as herein described in a sterile saline medium. Various administration techniques are possible, depending among other things on the size of the enzyme or polypeptide fragment. It needs to be borne in mind that it is difficult or impossible for many substances to pass into the brain from body fluids outside the brain. Administration may be intravenous, provided that the polypeptide is capable of crossing the blood brain barrier. Or administration may be into the cerebrospinal fluid outside the brain, for example in the lumbar region, using a depot preparation to exploit the characteristic long lasting action of the substance. Or administration can be effected directly into the ventricular cerebrospinal fluid, via an implanted cannula. This procedure is already established in several centres in North America for the administration of cholinergic drugs to patients with serve dementia relatively early in their life (Gauthier et al, 1986). Effective doses depend among other things on route of administration and are likely to be in the range of about 0.01 to 1000 mg, repeated as necessary. For example, a simple infusion of a polypeptide into human cerebrospinal fluid in a dose of the order of milligrams may produce effects lasting several days.

It is possible that AChE may exert its neuromodulatory action, not directly but indirectly by displaying trypsin-like proteolytic activity on some as-yet unidentified protein substrate in the brain. However, it is

4

more likely that the effects are exerted directly.

It can be predicted that this proposed therapy will more closely mimic normal brain function than traditional methods of treating Parkinsons disease and Alzheimers disease based upon transmitter replacement therapies. Traditional drugs acting on the brain all work by modifying particular neurotransmitter systems irrespective of brain area. The proposed therapy is different because (a) the biological activity is neuromodulatory and so will only be revealed when classical neurotransmitters are released by normal afferent neuronal activity within specific neuronal systems; (b) the substance will affect all the specific neuronal systems that are pathologically altered in the disease; (c) the effect will be more long-lasting than normal therapies, so requiring less frequent administration of the substance. Hence a more natural balance will be restored and other brain systems will be left relatively unaffected. For these reasons the side effects may be less pronounced than those of therapies based upon replacing just one transmitter.

## EXAMPLE 1

## SYNTHESIS AND CHARACTERISATION OF THE TRIPEPTIDE Pro-Glu-Pro

The tri-peptide was synthesised by sequentially coupling protected amino acids to a derivatised benzyl ester resin Boc Pro-O-Resin. The washing procedure used was:

## DEPROTECTION

1. Dichloromethane (DCM), 1 $\times$ 1 min:
2. 40% Trifluoroacetic Acid (TFA)/DCM, 1 $\times$ 1 min
3. 40% TFA/DCM, 1 $\times$ 10 minutes
4. DCM, 1 $\times$ 1 min:
5. Ethanol, 1 $\times$ 1 min
6. DCM, 2 $\times$ 1 min

## NEUTRALIZATION

7. Triethylamine (TEA) 10%/DCM 1 $\times$ 10 min
8. TEA 10%/DCM, 1 $\times$ 10 min.

## COUPLING

9. DCM, 3 $\times$ 1 min:
10. Boc-Amino Acid 3 equiv, + Dicyclohexylcarbodiimide (DCCI), 3 equiv, 1 $\times$ 60 min.

## PURIFICATION

The product was obtained by chromatography on reverse phase silica eluting with $H_2O$ (0.1% TFA) up to Acetronitrile/$H_2O$ (60/40) 0.1% TFA to afford the pure peptide.

This peptide was characterised as homogenous by the following:
1. Thin-layer chromatography of silica gel in two solvent systems.
2. Reverse phase high pressure liquid chromatography (HPLC).
3. Paper electrophoresis.

The peptide has a composition, by amino acid analysis, consistent with its predicted structure.

## THIN LAYER CHROMATOGRAPHY

Silica    $F_m$ (nBuOH:Pyr:HOAc:$H_2O$, 15:15:3:12)   Ninhydrin

Silica    1:1:1:1 (nBuOH:EtOAc:HOAc:$H_2O$)      Ninhydrin

Results   $F_m$ Single spot.     $R_f$ = 0.28

         1:1:1:1 Single spot   $R_f$ = 0.54

## ELECTROPHORESIS

Whatman 3MM, pH 1.9(HCOOH:Acet.); 1000V. 1hr o-tolidine
Results:
Single spot travelling towards the cathode.
$R_f$ = 0.52 with reference to Picric acid.

## AMINO ACID ANALYSIS

Chromatogram No. 63-4159      % of peptide 78.9%

|     | Theory | Found |
| --- | --- | --- |
| Glu | 1 | 0.98 |
| Pro | 2 | 2.01 |

HPLC Model Gilson
    Column Vydac C-18.
    Solvent A. 0.1% TFA; B. 60% $CH_3$ CN in A.
    Gradient 0% to 100% linear in 40 min
    Detector 210 nm 2 AUFS
Single sharp peak at 3.3 min. (8.5% B)

## EXAMPLE 2

## SYNTHESIS AND CHARACTERISATION OF THE TETRAPEPTIDE Arg-Pro-Glu-Pro

The tetrapeptide was synthesised by sequentially coupling protected amino acids to a derivatised hydroxy benzyl resin, Boc Pro-O-Resin (5.71g, 4mM). The washing procedure used was:

## DEPROTECTION

1. 1 × 40% Trifluoroacetic acid in dichloromethane (TFA/DCM) with indole scavenger (1 min).
2. 1 × TFA (30 min).
3. 1 × DCM (1 min).
4. 1 × Isopropanol (1 min).
5. 2 × DCM (1 min).

## NEUTRALIZATION

6. 1 × Triethylamine (TEA) 10% in DCM (1 min).
7. 1 × TEA (10 min).

## COUPLING

8. 3 × DCM (1 min).
9. 1 × Boc amino acid in DCM solvent with DCCI (dicyclohexylcarbodiimide) (12 mM), for up to 1 hour. The amino acids used were Boc Glu-OBzl (4.04g, 12 mM); Boc Pro (2.58g, 12 mM); and Boc Arg (Tos) (5.14g, 12 mM).

The resin was dried and cleaved by HF and lyophioised to give a crude product which was purified using reverse phase silica chromatography.

This peptide was characterised as homogenous by the following:

1. Thin-layer chromatography of silica gel in two solvent systems.
2. Reverse phase high pressure liquid chromatography (HPLC).
3. Paper electrophoresis.

The peptide has a composition, by amino acid analysis, consistent with its predicted structure.

## THIN LAYER CHROMATOGRAPHY

Silica   F (nBuOH:Pyr:HOAc:$H_2O$, 15:10:3:12)   Ninhydrin

Silica   1:1:1:1 (nBuOH:EtOAc:HOAc:$H_2O$)   Ninhydrin

Results   F Main spot with a faint lower spot   $R_f = 0.41$

1:1:1:1 Main spot with a

negligible upper spot.   $R_f = 0.50$

## ELECTROPHORESIS

Whatman 3MM, pH 1.9(HCOOH:Acet.); 1000V. 1hr o-tolidine
Results:
Single spot travelling toward the cathode
$R_f$ = 0.50 with reference to Picric acid

### AMINO ACID ANALYSIS

Chromatogram No. 63-6043   % of peptide 78.4%

| | Theory | Found |
|---|---|---|
| Glu | 1 | 1.02 |
| Pro | 2 | 1.98 |
| Arg | 1 | 1.00 |

HPLC Model Gilson
Column Vydac C-18.
Solvent A. 0.1% TFA; B. 60% $CH_3CN$ in A.
Gradient 0% to 100% linear in 40 min
Detector 210 nm 0.5 AUFS
Single sharp peak at 19.0 min. (47% B)

EXAMPLE 3

BIOLOGICAL ACTIVITY OF Pro-Glu-Pro

The possible biological actions of Pro-Glu-Pro are being examined on the membrane properties of nigrostriatal neurons in brain slices from guinea-pigs maintained in vitro. Slices of the midbrain are maintained in an oxygenated perfusion medium and the cell bodies of putative dopaminergic nigrostriatal neurons are impaled with a microelectrode in order to record voltage transients across the cell membrane (Nedergaard et al Exp. Brain Res. 69, 444-448, 1988). Pro-Glu-Pro is applied either via the medium superfusing the brain slice or by pressure ejection from a microcapillary directly into the tissue. The final bath concentration tested so far ranged from 2.5 microgram/ml to 20 microgram/ml. Concentrations of the peptide above 5 microgram/ml had actions similar to those reported for AChE (Greenfield, Jack, Last & French, Exp. Brain Res. 1988, in press), i.e. they caused a hyperpolarisation of the nerve cell's membrane. The effect was more rapid in onset and more rapidly reversible than with AChE. The ionic basis for this action of the peptide is being investigated to see if it is the same as that found for AChE (Greenfield et al. Exp. Brain Res. 1988 in press: as above). More "physiological" concentrations of the peptide will be tested by pressure injection.

These studies are being repeated with the tetrapeptide of Example 2.

References

Appleyard, M.E., A.D. Smith, P. Berman, G.K. Wilcock, Esiri, M.M., D. Neary, and D.M. Bowen (1987) Cholinesterase activities in cerebrospinal fluid of patients with senile dementia of the Alzheimer type. Brain, Vol 110, P 1309-22.

Appleyard, M.E., A.D. Smith, G.K. Wilcock, and M.M. Esiri (1983) Decreased CSF acetylcholinesterase activity in Alzheimers disease. Lancet ii:452.

Chubb, I.W., S. Goodman, and A.D. Smith (1976) Is acetylcholinesterase secreted from central neurons into cerebrospinal fluid? Neuroscience 1:57-62.

Cuello, A.C., E. Romero, and A.D. Smith (1981) In vitro release of acetylcholinesterase from the rat substantia nigra. J. Physiol. 312:14P-15P

Gauthier, S. et a. (1986) Transmitter-replacement therapy in alzheimers disease using intracerebroventricular infusions of receptors agonists. Can. J. Neurol. Sci 13:394.

Greenfield, S.A. (1985) Review: The significance of dendritic release of transmitter and protein in the substantia nigra. Neurochem. Internat. 7:887-901.

Greenfield, S.A., M.E. Appleyard, and M.R. Bloomfield (1986) 6-hydroxydopamine-induced turning behavior in the rat: the significance of acetylcholinesterase in cerebrospinal fluid. Behav. Brain Res. 21:47-54.

Greenfield, S.A., W.I. Chubb, R.A. Grunewald, Z. Henderson, J. May, S. Portnoy, J. Weston, and M.C. Wright (1984) A non-cholinergic function for acetylcholinesterase in the substantia nigra: Behavioural evidence. Exp. Brain Res. 54:513-520.

Greenfield, S.A. J.J.B. Jack, A.T.J. Last, and M. French (1987) An electrophysiological action of acetylcholinesterase indpendent of its catalytic site. Exp. Brain Res., in press.

Greenfield, S.A. and A.D. Smith (1979) the influence of electrical stimulation of certain brain areas on the concentration of acetylcholinesterase in rabbit cerebrospinal fluid. Brain Res. 177:445-459.

Greenfield, S.A., A. Cheramy, V. Leviel, and J. Glowinski (1980) In vivo release of acetylcholinesterase in cat substantia nigra and caudate nucleus. Nature 284:355-357.

Last, A.T.J. and S.A. Greenfield (1987) Acetylcholinesterase has a non-cholinergic neuromodulatory action in the guinea-pig substantia nigra. Exp. Brain Res., Vol 67, P.445-8.

Loh, Y.P., M.J. Brownstein, and H. Gainer (1984) Proteolysis in neuropeptide process and other neural functions. A. Rev. Neurosci. 7:189-222.

Massoulie, J. and S. Bon (1982) The molecular forms of cholinesterase and acetylcholinesterase in vertebrates. A. Rev. Neurosci. 5:57-106.

Nakamura, S. and S.R. Vincent (1985) Acetylcholinesterase. and somatostatin-immunoreactivity coexist in human neocortex. Neurosci. Lett. 61:183-187.

Ralston, J.S., R.S. Rush, B.P. Doctor, and D. wolfe (1985) Acetylcholinesterase from fetal bovine serum, purification and characterisation of soluble G4 enzyme. J.Biol. chem. 260:4312-4318.

Schumacher, M., S. Camp, Y. Maulet, M. Newton, K. MacPhee-Quiqley, S.S. Taylor, T. Friedmann, and P. Taylor (1986) Primary structure of Torpedo californica acetylcholinesterase deduced from its cDNA sequence. Nature 319:407-409.

Small, D.H., Z. Ismael, and I.W. Chubb (1986) Acetylcholinesterase hydrolyses chromogranin A to yield low molecular weight peptides. Neuroscience 19:289-295.

Smith, A.D. and A.C. Cuello (1984) Alzheimers disease and acetylcholinesterase-containing neurons. Lancent ii:513.

Vogt, M., A.D. Smith, and L.D.Fuenmayor (1984) Factors influencing the cholinesterases of cerebrospinal fluid in the anaesthetized cat. Neuroscience 12:979-995.

Weston, J. and S.A. Greenfield (1985) Application of acetylcholinesterase to the substantia nigra induces stereotypy in rats. Behav. Brain Res. 18:71-74.

Weston, J. and S.A. Greenfield (1986) Release of acetylcholinesterase in the rat nigrostriatal pathway; relation to receptor activation and firing rate. neuroscience 17:1079-1088.

Zevin-Sonkin, D., A. Avni, R. Zisling, R. Koch, and H. Soreq (1985) Expressions of acetylcholinesterase gene(s) in the human brain - molecular cloning evidence for cross-homologous sequences. J. Physiol. Paris 80:221-228.

## Claims

1. A substance for the treatment of disorders of the central nervous system, which substance is acetylcholinesterase (AChE) or a variant or fragment thereof having the same biological actions.

2. A substance as claimed in claim 1, wherein the AChE or variant or fragment thereof shows neuromodulatory activity.

3. A substance as claimed in claim 2, wherein the neuromodulatory activity is not confined to cholinergic systems.

4. The tripeptide Pro-Glu-Pro, in which the carboxylic acid group of Glu may be free, esterified or amidated.

5. A polypeptide having the following amino acid sequence, or a conservative variant thereof, any carboxylic acid group being optionally esterified or amidated:-

Pro-Trp-Ser-Gly-Val-Trp-Asn-Ala-Ser-Thr-Thr-Pro-Asn-Asn-Cys-Gln-Gln-Tyr-Val-Asp-Glu-Gln-Phe-Pro-Gly-Phe-Ser-Gly-Ser-Glu-Met-Trp-Asn-Pro-Asn-Arg-Glu-Met-Ser-Glu-Asp-Cys-Leu-Tyr-Leu-Asn-Ile-Trp-Val-Pro-Ser-Pro-Arg-Pro-Lys-Ser-Thr-Thr-Val-Met-Val-Trp-Ile-Tyr-Gly-Gly-Gly-Phe-Tyr-Ser-Gly-Ser-Ser-Thr-Leu-Asp-Val-Tyr-Asn-NH$_2$.

6. A polypeptide having the following amino acid sequence, or a conservative variant thereof, any carboxylic acid group being optionally esterified or amidated, the sequence optionally being extended at the carboxyl end by Gly:-

Ala-Ile-Leu-Gln-Ser-Gly-Ser-Pro-Asn-Cys-Pro-Trp-Ala-Ser-Val-Ser-Val-Ala-Glu-NH$_2$.

7. A polypeptide having the following amino acid sequence, or a conservative variant thereof, any carboxylic acid group being optionally esterified or amidated:-

Ala-Val-Glu-Leu.

8. A polypeptide having the following amino acid sequence, or a conservative variant thereof, any carboxylic acid group being optionally esterified or amidated:-

Asn-Leu-Asn-Cys-Asn-Leu-Asn-Ser-Asp-Glu-Glu-Leu-Ile-His-Cys-Leu-Arg-Glu.

9. A polypeptide having the following amino acid sequence, or a conservative variant thereof, any carboxylic acid group being optionally esterified or amidated:-

Pro-Gln-Glu-Leu-Ile-Asp-Val-Glu-Trp-Asn-Val-Leu-Pro-Phe-Asp-Ser-Ile-Phe-Arg-Phe-Ser-Phe-Val-Pro-Val-Ile-Asp-Gly-Glu-Phe-Phe-Pro-Thr-Ser-Leu-Glu-Ser-Met-Leu-Asn-Ser-Gly-Asn-Phe.

10. A polypeptide having the following amino acid sequence, or a conservative variant thereof, any carboxylic acid group being optionally esterified or amidated, the sequence optionally being extended at the carboxyl end by Gly:-

Asp-Asp-His-Ser-Glu-Leu-Leu-Val-Asn-Thr-Lys-Ser-NH$_2$.

11. A polypeptide as claimed in any one of claims 4 to 10 in which there is present an additional basic amino acid residue, Lys or Arg, at either the carboxy terminal end or the amino terminal end, or both.

12. The tetrapeptide Arg-Pro-Glu-Pro, in which the carboxylic acid group of Glu may be free, esterfied or amidated.

13. A composition for administration to a patient suffering from a disorder of the central nervous system comprising a substance or polypeptide according to any one of claims 1 to 12 in a sterile saline medium.

14. A DNA sequence which codes for a polypeptide as claimed in any one of claims 4 to 12.